# EUROPÄISCHE PATENTSCHRIFT

(11) **EP 0 949 925 B1**
(45) Veröffentlichungstag und Bekanntmachung des Hinweises auf die Patenterteilung: **07.01.2004**
(21) Anmeldenummer: 97952767.8
(22) Anmeldetag: 14.11.1997
(51) Int. Cl.: A61K 31/485, A61K 9/70

(54) **FLACHE ARZNEIZUBEREITUNG ZUR APPLIKATION UND FREISETZUNG VON BUPRENORPHIN ODER EINER PHARMAKOLOGISCH VERGLEICHBAREN SUBSTANZ IN DER MUNDHÖHLE UND VERFAHREN ZU IHRER HERSTELLUNG**
FLAT MEDICAMENT PREPARATION FOR THE APPLICATION AND RELEASE OF BUPRENORPHINE OR A PHARMACOLOGICALLY COMPARABLE SUBSTANCE IN THE BUCCAL CAVITY, AND METHOD OF PRODUCING THE SAME
PREPARATION MEDICAMENTEUSE PLATE POUR ADMINISTRER OU LIBERER, DANS LA CAVITE BUCCALE, DE LA BUPRENORPHINE OU UNE SUBSTANCE COMPARABLE SUR LE PLAN PHARMACOLOGIQUE ET PROCEDE PERMETTANT DE LA PREPARER

(30) Priorität: 16.12.1996 DE 19652188
(43) Veröffentlichungstag der Anmeldung: 20.10.1999
(73) Patentinhaber: LTS LOHMANN Therapie-Systeme AG, 56626 Andernach (DE)
(72) Erfinder: CREMER, Karsten, D-53119 Bonn (DE); LUESSEN, Henrik, D-56579 Rengsdorf (DE)
(74) Vertreter: Flaccus, Rolf-Dieter, Dr.
(86) Internationale Anmeldenummer: PCT/EP1997/006369
(87) Internationale Veröffentlichungsnummer: WO 1998/026780

(56) Entgegenhaltungen:
- EP-A- 0 219 762
- FR-A- 2 514 642
- US-A- 4 673 679
- J.P. CASSIDY ET AL.: "controlled buccal delivery of buprenorphine" JOURNAL OF CONTROLLED RELEASE, Bd. 25, Nr. 1/2, 27.Mai 1993, AMSTERDAM (NL), Seiten 21-29, XP000361365

## Beschreibung

Vorliegende Erfindung betrifft eine Arneizubereitung zur Applikation von Buprenorphin oder pharmakologisch vergleichbaren Wirkstoffen im Bereich der Mundhöhle bzw. der Mundschleimhaut. Sie betrifft insbesondere eine Zubereitung, die flach und als folien-, papier- oder oblatenartige Darreichungsform ausgestaltet ist.

Flache Wirkstoffträger wurden bereits für verschiedene Zwecke entwickelt und hergestellt. Als grundlegend für diese Darreichungsform kann die DE-OS 27 46 414 angesehen werden, die ein folienartiges Band aus Wirkstoff, Bindemittel und weiteren Hilfsstoffen beschreibt, bei dem aufgrund homogenen Dicke, Dichte und Breite ein direkter Zusammenhang zwischen einer Längeneinheit des Bandes und der darin enthaltenen Wirkstoffdosis besteht. Die Vorteile der kontinuierlichen Dosierbarkeit wurden auch von anderen Anmeldern erkannt und in speziellen Einzelvarianten beschrieben. So beschreibt DE-PS 36 30 603 ein flächiges Trägermaterial z.B. in Form eines Trennpapieres mit einer wirkstoffhaltigen Beschichtung, wobei letztere nach Vorzerteilung in Dosiereinheiten vom Trägermaterial dosisweise abziehbar ist.

Die Praktikabilität des flachen Formates im allgemeinen sowie die Vorteile bei der Herstellung der Darreichungsform und bei der Dosierung unter ihrer Anwendung wurden im Stand der Technik erkannt. Darüber hinaus lassen sich weitere Vorteile solcher Darreichungsformen ableiten, wie etwa die Bedruckbarkeit einer relativ großen Fläche auf der Arzneiform im Verhältnis zu ihrem Gewicht, womit die Einnahmesicherheit erhöht werden kann, wie auch die Möglichkeit der diskreten Einnahme, ohne daß Flüssigkeit zur Verfügung steht.

Trotz dieser klaren Vorteile haben sich solche Darreichungsformen bisher kaum durchgesetzt. Offensichtlich reicht für viele Hersteller von Pharmazeutika der Nutzen gegenüber konventionellen Darreichungsformen nicht aus, um Produkte dieser Art mit den gebräuchlichen Wirkstoffen zu entwickeln und deren arzneimittelrechtliche Zulassung zu betreiben. Darüber hinaus können vorhandene Produktionsmaschinen und existierendes Knowhow für diese neuartigen Produkte nicht genutzt werden; ein hoher Investitionsbedari: würde entstehen. Trotz der oben beschriebenen Vorteile von flächen- film- oder papierartigen Darreichungsformen ist der therapeutische und/oder wirtschaftliche Nutzen bei der Verabreichung von gängigen, auch peroral applizierbaren Wirkstoffen im Vergleich zu konventionellen Tabletten anscheinend nicht so groß, daß er die Kosten der Umstellung auf diese Darreichungsformen rechtfertigen würde.

Zu den Wirkstoffen, dies sich nur wenig für eine perorale Verabreichung eignen, zählt das in der Schmerztherapie seit Jahren erfolgreich eingesetzte Opiat Buprenorphin. Nach peroraler Applikation ist es kaum bioverfügbar, d. h. erscheint nur in einem sehr geringen Ausmaß von wenigen Prozent der eingenommenen Dosis im Blutkreislauf (McQuay & Moore, in: Buprenorphine, Hrsg. Cowan & Lewis, New York 1995). Der Grund für die mangelnde Bioverfügbarkeit liegt vermutlich im weitgehenden Abbau der Substanz während der ersten Leberpassage nach der gastrointestinalen Resorption ("First-pass Effekt"). Eine Möglichkeit, den First-pass-Effekt bei der oralen Verabreichung zu umgehen, besteht darin, den Wirkstoff bereits an der Mundschleimhaut zur Resorption zu bringen. Wirkstoff, welcher hier ins Blut übertritt, muß nicht als erstes das Pfortadersystem und damit in konzentrierter Form die den Wirkstoff metabolisierende Leber passieren, um in den zentralen Körperkreislauf zu gelangen. Voraussetzung für eine buccale oder sublinguale Applikation ist jedoch die ausreichende Permeabilität der oralen Mucosa für den Wirkstoff unter Berücksichtigung der notwendigen Dosis. Die Permeabilität wiederum hängt in hohem Maße von den physikochemischen Eigenschaften des Wirkstoffs ab. Da Buprenorphin in sehr geringen Dosen wirksam ist und außerdem die erforderlichen physikochemischen Charakteristika besitzt, ist die buccale oder sublinguale Applikation sehr attraktiv.

In der Zeitschrift "Journal of Controlled Release", Bd. 25, Nr. 1/2, 1993, werden nicht zerfallsfähige polymere Systeme zur buccalen Anwendung von Buprenorphin beschrieben, die mit einer für den Wirkstoff nicht permeablen Schicht abgedeckt wurden, so daß eine gerichtete Freisetzung des Wirkstoffs möglich und der Verlust von Wirkstoff durch Schlucken vermindert wurde. Die wirkstoffundurchlässige Schicht wurde dabei mit Hilfe eines randständig aufgebrachten Klebers in der Mundhöhle in Position gehalten und das System wurde nach mehreren Stunden wieder aus der Mundhöhle entfernt.

Die US Patentschrift US 4 673 679 offenbart eine pharmazeutische Zubereitung zur buccalen und sublingualen Verabreichung von Opiaten und Opiatantagonisten in Form von im wässrigen Medium der Mundhöhle zerfallsfähigen Pflastern. Die zur buccalen Anwendung offenbarten Pflaster enthalten u. a. den mukoadhäsiven Hilfsstoff Carbopol 934P sowie ersterifizierte Derivate von Nalbuphin als "prodrugs".

Tatsächlich befinden sich - zumindest in Deutschland - neben den injektabilen Darreichungsformen keine peroralen, sondern nur sog. Sublingualtabletten mit Buprenorphin im Handel (Temgesic® sublingual). Diese Tabletten würdigen zwar - wenn auch vorwiegend durch die Einnahmevorschrift, denn nur diese, nicht die Tablette an sich, legt die sublinguale Gabe nahe - die Tatsache, daß eine sublinguale Applikation des Wirkstoffes der peroralen vorzuziehen ist; sie bieten jedoch ein für den Anwendungszweck mit erhetblichen Nachteilen behaftetes Vehikel. Hierzu gehört zunächst die nicht unbeträchtliche Zerfallzeit, die bei gepreßten Tabletten selbst unter günstigen Voraussetzungen mindestens einige Minuten beträgt, bei den im Handel erhältlichen Buprenorphin-Tabletten in der Regel etwa 5 bis 10 Minuten. Für Patienten mit starken, akuten Schmerzen bedeutet diese Zerfallzeit eine unerwünschte Verzögerung des Wirkstoffeintritts, bei einer Substitutions- oder Entwöhnungstherapie dagegen eine zeitliche Belastung des medizinischen Personals, welches die bestimmungsgemäße Verwendung der Tabletten überwachen und eine mißbräuchliche Wiederentnahme der unzerfallenen Tabletten aus dem Mund verhindern muß. Weitere Nachteile der Tablette sind das Fremdkörpergefühl im Mund während der Zerfallzeit, aber auch die große Variabilität beim Ausmaß der sublingualen Absorption, die dadurch verursacht wird, daß der Wirkstoff beim oder nach dem Zerfall der Tablette überwiegend keinen direkten Kontakt zur Mundschleimhaut hat, sondern in den Speichel freigesetzt wird; der Speichel kann sich aber mehr und weniger zufällig über eine sehr variable Zeit in der Mundhöhle befinden, bevor er geschluckt wird.

Aufgabe der vorliegenden Erfindung ist daher die Schaffung von Arzneizubereitungen auf der Basis von und mit den allgemeinen Vorteilen von flachen, film- oder papierartigen Wirkstoffträgern, welche durch die Kombination mit einem speziellen Wirkstoff noch zusätzliche therapeutische und/oder wirtschaftliche Vorteile gegenüber Arzneizubereitungen desselben Wirkstoffes auf der Basis konventioneller Darreichungsformen wie etwa Tabletten aufweist. Darüber hinaus ist es ebenso die Aufgabe der Erfindung, eine Applikationsform für Buprenorphin bereitzustellen, die den Wirkstoff in der Mundhöhle freisetzt, ohne die im Stand der Technik beschriebenen Nachteile zu besitzen.

Die Aufgabe wird entsprechend den Merkmalen des Anspruchs 1 dadurch gelöst, dass eine im wässrigen Medium der Mundhöhle zerfallsfähige oblatenförmige Arzneizubereitung mit einer mukoadhäsiven, als Wirkstoff Buprenorphin oder eine therapeutisch vergleichbare Wirksubstanz enthaltende Schicht auf der Basis von wasserlöslichen filmbildenden Polymeren bereitgestellt wird, die eine der mucoadhäsiven Fläche entgegengesetzte nicht-mucoadhäsive Außenschicht mit geringerer Permeabilität für den Wirkstoff aufweist.

Eine Arzneizubereitung nach Anspruch 1 ist, wie im folgenden dargelegt werden soll, einer konventionellen Darreichungsform zur Verabreichung von Buprenorphin sowohl unter wirtschaftlichen als auch unter therapeutischen Gesichtspunkten weit überlegen und eignet sich insbesondere einerseits zur Analgesie bei starken Schmerzzuständen, andererseits zur Therapie der Opiat oder Cocainabhängigkeit im Sinne einer Substitutionstherapie oder eines Entwöhnungsprogrammes.

Die Arzneizubereitung nach Anspruch 1 kann bei der Applikation direkt mit der Mundschleimhaut in Kontakt gebracht werden. Durch die flächige Ausgestaltung befindet sich sofort nach der Applikation etwa die Hälfte der ohnehin großen Oberfläche der Darreichungsform unmittelbar auf der Mucosa. Das freigesetzte Buprenorphin findet also für den Eintritt in den Körper zwei besonders günstige Faktoren vor, nämlich eine kurze Diffusionsstrecke und eine große Diffusionsfläche. Hierdurch wird der Anteil an Buprenorphin herabgesetzt, der verschluckt wird, was bei vielen anderen Wirkstoffen nicht sonderlich problematisch wäre. Bei Buprenorphin jedoch ist das Verschlucken von Wirkstoff möglichst zu vermeiden oder herabzusetzen, da verschlucktes Buprenorphin aus den dargelegten Gründen unwirksam bleibt. Bereits bei der einfachsten erfindungsgemäßen Ausgestaltung und mit einer Zerfallzeit von wenigen Minuten nach Applikation oder nach dem Einbringen in wässrige Medien wird sich daher die Überlegenheit eines buprehorphinhaltigen Films gegenüber einer buprenorphinhaltigen Tablette zeigen.

Ein verbesserter Kontakt der erfindungsgemäßen Arzneizubereitung mit der Mundschleimhaut läßt sich durch die Auswahl der Hilfsstoffe herbeiführen. Von bestimmten pharmazeutisch gebräuchlichen, oral applizierbaren Hilfsstoffen ist bekannt, daß sie schleimhauthaftende Eigenschaften besitzen. Beispiele für solche mucoadhäsiven Substanzen sind Polyacrylsäure, Carboxymethylcellulose, Traganth, Alginsäure, Gelatine, Hydroxymethylcellulose,Methylcellulose und Gummi Arabicum. Darüber hinaus ist von verschiedenen nicht mucoadhäsiven Stoffen bekannt, daß sie in bestimmten Mischungsverhältnissen ebenfalls mucoadhäsive Eigenschaften ausbilden. Ein Beispiel für ein solches Gemisch ist Glycerinmonooleat/ Wasser im Verhältnis 84:16 (Engström et. aL., Pharm. Tech. Eur. 7 [1995], Nr. 2, S. 14-17).

Durch den zwei oder mehrschichtigen Aufbau der Darreichungsform der erfindungsgemäßen Arzneizubereitung kann vermieden werden, daß die Zubereitung verschiedene Schleimhautpartien miteinander verklebt, was zu erheblichen Missempfindungen führen würde. Außerdem kann bei einem solchen Aufbau, bei dem die nicht mukoadhäsive Schicht eine relativ geringere Permeabilität gegenüber dem Wirkstoff als die mukoadhäsive Schicht besitzt, vermieden werden, dass durch die Freisetzung in den Speichel der Mundhöhle statt zur Schleimhaut Wirkstoffverluste eintreten.

Erfindungsgemäße Arzneizuberoitungen sind auch solche, die neben dem Wirkstoff Buprenorphin oder einem diesem pharmakologisch vergleichbaren Wirkstoff noch einen oder mehrere weitere Wirkstoffe enthalten. Eine solche Zubereitung kann in mehrfacher Hinsicht vorteilhaft sein. Zum einen ist es eine anerkannte Methode zur Behandlung mehrerer gleichzeitig auftretender Symptome oder Zustände, eine fixe Wirkstoffkombination in einem Medikament zu verabreichen. Hierzu lassen sich beliebige, therapeutisch sinnvolle Wirkstoffe in die erfindungsgemäße Zubereitung einarbeiten. Zum anderen ist die erfindungsgemäße Kombination eines Opiatwirkstoffes mit einer anderen Substanz, welche die spezifischen Risiken einer Opiatverabreichung reduzieren kann, besonders sinnvoll und vorteilhaft. So lassen sich beispielsweise - gegebenenfalls partielle- Opiatantagonisten wie etwa Nalbuphin, Naloxon oder Naltrexon mit dem Opiatwirkstoff kombinieren, was zur Folge hat, daß die Sucht- bzw. Gewöhnungsgefahr durch die wiederholte Verabreichung der Zubereitung dadurch verringert wird, daß sich die Dosis nicht steigern läßt, ohne gleichzeitig eine Steigerung des antagonistischen Effektes in Kauf zu nehmen. Von der Wahl eines geeigneten Antagonisten sowie des Dosisverhältnisses in der Zubereitung wird der Erfolg dieser Strategie abhängen.

Wenn auch Buprenorphin - gegebenenfalls in Form eines seiner therapeutisch akzeptablen Salze - der am meisten bevorzugte Wirkstoff ist, betrifft die Erfindung auch solche Wirkstoffe, die dem Buprenorphin pharmakologisch ähnlich oder vergleichbar sind, da die beschriebenen Vorteile der Erfindung, wenn auch in unterschiedlichem Ausmaße, auch hier zum Tragen kommen können. Insbesondere sind weitere geeignete Wirkstoffe, die hier auch als "pharmakologisch ähnlich oder vergleichbar " bezeichnet sind, solche, die den Opiaten oder Opioiden zuzurechnen sind, da viele von ihnen nicht nur pharmakodynamisch, sondern auch pharmakokinetische Ähnlichkeiten mit Buprenorphin aufweisen, also eine relativ niedrige Dosis, eine gute Membrangängigkeit und einen hohen First-Pass-Effekt. Insbesondere bevorzugt sind Morphin- oder Dihydromorphinderivate sowie Substanzen aus der Methadon und aus der Fentanylgruppe.

Um einer mißbräuchlichen oder nicht bestimmungsgemäßen Anwendung keinen Vorschub zu leisten, wird die erfindungsgemäße Arzneizubereitung in der Regel dosisweise vorzerteilt und voneinander separiert in einer geeigneten Verpackung vorliegen, so daß zur Entnahme einer Dosiseinheit jeweils nur diese entnehmbar gemacht wird, wie etwa im Falle einer Blisterpackung, in welcher jede Dosiseinheit in einem Tiefziehnapf einzeln eingesiegelt ist. Im Rahmen von Programmen zur Behandlung der Opiatoder Cocainabhängigkeit kann es jedoch auch sinnvoll sein, z. B. den betreuenden Ärzten die Zubereitung in Form von Verpackungseinheiton anzubieten, in denen sie als unzerteiltes blatt- oder bandförmiges Material vorliegt, von welchem sich die Dosiseinheiten zum Zwecke der Applikation abteilen lassen. Dies erleichtert eine Massenapplikation und gibt den verabreichenden Ärzten die Möglichkeit, unterschiedliche Dosiseinheiten je nach Dosisbedarf aus ein und demselben Material abzuteilen.

Da von der erfindungsgemäßen Arzneizubereitung ein gegenüber bekannten Zubereitungen erhöhtes Ausmaß der Bioverfügbarkeit zu erwarten ist, muß die Dosierung gegebenenfalls angepaßt werden. Im Falle des Buprenorphins wird die analgetische Einzeldosis bei 0,1 bis 1 mg liegen, in der Suchttherapie bzw. Substitutionstherapie jedoch möglicherweise deutlich höher.
Die Herstellung der Arzneizubereitung erfolgt erfindungsgemäß in mehreren Schritten. Zur Herstellung des bahnförmigen Ausgangsmaterials, aus dem zuletzt entweder die Einzeldosen oder aber ganze Verpackungseinheiten durch Schneiden oder Stanzen abgeteilt werden, sind zwei grundlegende Verfahrensvarianten geeignet. Die erste Gruppe von Verfahren umfaßt jene, bei denen mit wässrigen bzw. lösemittelhaltigen Flüssigkeiten teilweise höherer Viskoität ein Band oder eine Prozessfolie gleichmäßig beschichtet und anschließend einem Trocknungsprozeß unterworfen wird. Hierzu wird zunächst die Beschichtungsmasse hergestellt, wozu mindestens ein wasserlösliches, zur Filmbildung befähigtes Polymer, der oder die Wirkstoffe und eine geeignete, verdampfbare Flüssigkeit innig gemischt werden müssen. Bedarfweise können weitere Hilfsstoffe wie zerfallmodifizierende Polymere, Weichmacher, Füllstoffe, texturvermittelnde Substanzen, Pigmente, Farbstoffe, Geschmackkorrigenzien, Löslichkeitsvermittler, Substanzen zur Einstellung des pH-Wertes, Glättungsmittel, Mattierungsmittel, Zerfallbeschleuniger etc. eingearbeitet werden. Alternativ läßt sich das bahnförmige Ausgangsmaterial durch thermoplastische Formung, d. h. ohne Zuhilfenahme von Flüssigkeiten herstellen. Hierzu gehören alle Hot-Melt-Beschichtungs- und alle Extrusionsverfahren. Eine Voraussetzung ist in diesem Fall, daß das zur Filmbildung befähigte Polymer oder Polymergemisch thermoplastisch formbar ist. Die erforderlichen Zutaten werden gemischt und unter Einwirkung von Druck und/oder Wärme durch Extrudieren, Blasen oder durch Beschichten von Bändern oder Folien geformt und nach dem Erstarren der weiteren Verarbeitung zugeführt. Für die Herstellung von erfindungsgemäßen Zubereitungen mit mehrschichtigem Aufbau eignen sich entsprechend modifizierte Verfahren, wobei es unerheblich ist, ob mehrere bahnförmige Materialien gleichzeitig oder nacheinander hergestellt und zusammengefügt werden.

## Patentansprüche

1. Buccale Arzneizubereitung zur Bekämpfung starker Schmerzzustände oder Suchttherapie mit Buprenorphin, Morphin-, Dihydromorphin-Derivaten, Substanzen aus der Methadon- oder Fentanylgruppe oder als therapeutisch geeignetes Salz als Wirkstoff, **gekennzeichnet durch** eine im wäßrigen Medium der Mundhöhle zerfallfähige oblatenförmige Darreichungsform mit einer mucoadhäsiven, wirkstoffhaltigen Schicht auf der Basis von wasserlöslichen, filmbildenden Polymeren für die rasche Wirkstoffübertragung **durch** kurze Diffusionswege bei einer der wirksamen Dosis angemessenen großen Fläche, wobei die Darreichungsform eine der mucoadhäsiven Fläche entgegengesetzte nicht-mucoadhäsive Außenschicht mit geringerer Permeabilität für den Wirkstoff aufweist.

2. Arzneizubereitung nach Anspruch 1, **gekennzeichnet durch** einen zwei oder mehrschichtigen Aufbau mit einer mucoadhäsiven wirkstoffhlatigen Schicht auf der Basis von wasserlöslichen, filmbildenden Polymeren für die rasche Wirkstoffaufnahme **durch** kurze Diffusionswege.

3. Arzneizubereitung nach Anspruch 1 oder 2, **gekennzeichnet durch** einen Einzeldosen-Buprenorphingehalt von 0,1-1 mg.

4. Arzneizubereitung nach einem der vorangehenden Ansprüche, **dadurch gekennzeichnet, daß** sie durch den Zusatz eines haftungsvermittelnden Hilfsstoffes oder Hilfsstoffgemisches mit bio- bzw. mucoadhäsiven Eigenschaften ausgerüstet ist.

5. Arzneizubereitung nach Anspruch 4, **dadurch gekennzeichnet, daß** als weiterer Wirkstoff ein Opiatantagonist oder partieller Opiatantagonist vorhanden ist.

## Claims

1. Buccal pharmaceutical preparation for treating acute conditions of pain or for addiction therapy, comprising as active substance buprenorphine, morphine, dihydromorphine derivatives, substances from the methadone or fentanyl groups as such or as a therapeutically suitable salt, **characterized by** a wafer-shaped administration form, disintegratable in the aqueous medium of the oral cavity, which has a mucoadhesive, active substance-containing layer based on water-soluble, film-forming polymers, for rapid active substance transfer through short diffusion paths, while having a large surface appropriate to the effective dose, the said administration form having a non-muco-adhesive outer layer, opposed to the mucoadhesive surface, which outer layer has a lower permeability to the active substance.

2. Pharmaceutical preparation according to claim 1, **characterized by** a two- or multi-layered structure having a mucoadhesive active substance-containing layer based on water-soluble, film-forming polymers for rapid active substance uptake through short diffusion paths.

3. Pharmaceutical preparation according to claim 1 or 2, **characterized by** a single-dose buprenorphine content of 0.1-1 mg.

4. Pharmaceutical preparation according to any one of the preceding claims, **characterized in that** it is equipped with bioadhesive or mucoadhesive properties by the addition of an adhesion-promoting auxiliary substance or auxiliary substance mixture.

5. Pharmaceutical preparation according to claim 4, **characterized in that** as a further active substance an opiate or a partial opiate antagonist is present.

## Revendications

1. Préparation médicamenteuse destinée à être libérée dans la cavité buccale et à soulager des douleurs intenses ou à traiter une toxicomanie, qui comprend, en tant que principe actif, de la buprénorphine, des dérivés de la morphine et de la dihydromorphine, des substances du groupe de la méthadone ou du fentanyle, ou leurs sels thérapeutiquement appropriés, **caractérisée par** une forme galénique de type cachet apte à se désintégrer dans le milieu aqueux de la cavité buccale, qui porte une couche mucoadhésive contenant le principe actif, à base de polymères filmogènes hydrosolubles, permettant un transfert rapide du principe actif par un court trajet de diffusion, avec une surface dont les dimensions sont ajustées en fonction de la dose efficace, la forme galénique présentant une couche extérieure non-mucoadhésive, opposée à la surface mucoadhésive et moins perméable au principe actif.

2. Préparation médicamenteuse selon la revendication 1, **caractérisée par** une structure bicouche ou multicouche qui comprend une couche mucoadhésive contenant le principe actif, à base de polymères filmogènes hydrosolubles, permettant un transfert rapide du principe actif par un court trajet de diffusion.

3. Préparation médicamenteuse selon la revendication 1 ou 2, **caractérisée par** une teneur en buprénorphine correspondant à une dose individuelle de 0,1-1 mg.

4. Préparation médicamenteuse selon l'une quelconque des revendications précédentes, **caractérisée en ce que**, par suite de l'addition d'un adjuvant ou d'un mélange d'adjuvants favorisant l'adhérence, elle présente des propriétés bio- et/ou mucoadhésives.

5. Préparation médicamenteuse selon la revendication 4, **caractérisée en ce que** comme principe actif supplémentaire, elle contient un antagoniste des opiacés ou un antagoniste partiel des opiacés.
